# EUROPÄISCHE PATENTANMELDUNG

(11) **EP 0 834 742 A1**
(43) Veröffentlichungstag der Anmeldung: **08.04.1998**
(21) Anmeldenummer: 96115688.2
(22) Anmeldetag: 01.10.1996
(51) Int. Cl.: G01N 33/569, G01N 33/554

(54) **Spezifischer Anti-EBNA-1-Test mittels antikomplementärer Immunfluoreszenz**

(71) Anmelder: Gull Laboratories GmbH, 61343 Bad Homburg v.d.H. (DE)
(72) Erfinder: Bauer, Georg, Prof.Dr.rer.nat., 79104 Freiburg (DE)
(74) Vertreter: Cohausz & Florack

(57) **Zusammenfassung**

Verfahren zum spezifischen Nachweis von Antikörpern gegen EBNA-1 im Serum mittels Antikomplement-Immunfluoreszenz, indem zu EBNA-1 exprimierenden Zellen, die eine Deletion des EBNA-2 Gens tragen, ein Überschuß EBV negativer Humanzellen hinzugefügt und dieses Gemisch mit dem zu prüfenden Serum und Komplement behandelt und anschließend mit Fluorescein markiertem Antikomplement in Kontakt gebracht wird und auf diese Weise einen falschpositiven Befund vermeidet.

## Beschreibung

Das Epstein-Barr-Virus (EBV) ist ein menschliches Herpesvirus, das B-Lymphozyten und epitheliale Zellen infizieren kann. Seine Fähigkeit zu Persistenz und Latenz sowie seine proliferationsstimulierende und immortalisierende Wirkung auf B-Lymphozyten sind die Grundlage für die Pathogenese der EBV-verursachten Erkrankungen.

Die Primärinfektion mit EBV kann klinisch inapparent verlaufen oder zum Krankheitsbild der infektösen Mononukleose führen. Darüber hinaus ist EBV an der Entstehung bestimmter Tumore wie dem Burkitt-Lymphom, dem Nasopharynxkarzinom und dem Hodgkin-Lymphom beteiligt. Bei massiv Immunsupprimierten verseucht EBV polyklonale B-Zell-Lymphome, die sich zu einem monoklonalen Tumor weiterentwicklen können.

Die Diagnostik der EBV-Infektion, d. h. der Ausschuß oder Beweis einer akuten EBV-Infektion ist differentialdiagnostisch von großer Bedeutung. So gilt es, eine EBV-Infektion von anderen Erkrankungen, die mit einer Lymphadenopathie einhergehen, wie Toxoplasmose, CMV-Infektion, Röteln und Brucellose zu unterscheiden. Aufgrund der Begleithepatitis bei einer klinisch manifesten EBV-Infektion muß eine Abgrenzung zu Infektionen mit klassischen Hepatitiserregern, wie HAV, HBV und HCV sowie zu CMV erfolgen. Die neurologischen Symptome, die bei einer EBV-Primärinfektion auftreten können, erfordern eine Abgrenzung zu einer Vielzahl von neurotropen Viren. Schließlich kann das für die klinisch manifeste EBV-Primärinfektion typische Blutbild als Leukämie mißgedeutet werden. Auch hier ist eine sichere und eindeutige EBV-Serologie von großer Bedeutung.

Die Bedeutung der klassischen Marker der EBV-Serologie und deren Variabilität sind in den letzten Jahren sehr eingehend untersucht worden. So haben sich die klassischen Marker VCA-IgG und VCA-IgM als nicht ausreichend erwiesen, da VCA-IgM, das als Zeichen für eine frische EBV-Infektion gewertet wird, einerseits bei frischen Infektionen fehlen kann (falscher Ausschluß einer akuten EBV-Infektion) oder andererseits auch viele Monate bis Jahre nach einer akuten Infektion persistieren kann (falscher Nachweis einer akuten Infektion). Daher ist die zusätzliche Bestimmung der Antikörper gegen EBNA (Epstein-Barr-Virus-spezifische nukleäre Antigene) als zusätzlicher Marker in die EBV-Serologie aufgenommen worden.

Es wurde gezeigt, daß die spezifische Bestimmung Anti-EBNA-1 von zentraler Bedeutung für die EBV-Serologie ist. Anti-EBNA-1 ist in den ersten Wochen nach akuter EBV-Infektion regelmäßig negativ und erreicht nach Monaten positive Werte. Ein positives Anti-EBNA-1 ist daher ein spezifischer Hinweis, eine akute EBV-Infektion auszuschließen. Ein negatives Anti-EBNA-1 zusammen mit einem VCA-IgG von niedriger Avidität ist ein klarer Hinweis auf eine akute EBV-Infektion.

Die bisherige Anti-EBNA-Bestimmung mit Hilfe der Immunfluoreszenz erfolgt nach der von Reedman und Klein, Int. J. Cancer, 1973; 2: 499-520 für Gesamt-EBNA beschriebenen Technik der antikomplementären Immunfluoreszenz (ACIF). Dabei werden EBV-Genom enthaltende B-Lymphomzellen, die EBNA-Antigene exprimieren, fixiert. Der Nachweis beruht auf der Bindung EBNA-spezifischer Antikörper an den fixierten Zellen. An die gebundenen Antikörper wird Humankomplement angelagert, daran erfolgt Bindung mit Fluorescein markierter tierischer gegen das Komplement gerichteter Antikörper. Nach Anregung im Fluoreszenzmikroskop erfolgt ein Leuchten des Zellkernes, weil EBNA-Antigene nur im Kern anzutreffen sind. Da alle Zellen in der Population EBNA-positiv sind, leuchtet im Regelfall jede einzelne Zelle.

Zur Differenzierung zwischen Antikörpern gegen EBNA-1 oder EBNA-2 wurde in Kampmann et al., Med. Microbiol. Letters 1993; 2: 1-8 vorgeschlagen, einen bestimmten Subklon einer Zellinie EBV-positiver Burkitt-Lymphomzellen einzusetzen, die eine Deletion des EBNA-2 Gens tragen und somit nur EBNA-1 exprimieren können.

Neben EBNA-1 (100 % der Zellen) exprimieren etwa 5 % dieser Zellen VCA. Es ergibt sich also folgende Bewertung eines Testserums: Leuchtet im Test die Mehrzahl der Zellen, liegt ein positiver Anti-EBNA-1-Befund vor, der eine akute EBV-Infektion sicher ausschließt. In der Routinediagnostik liegt in 85 % der untersuchten Fälle dieser Befund vor; entsprechend ist seine Spezifität von großer Bedeutung. Leuchten nur 5 % der Zellen oder weniger, liegt kein spezifischer Anti-EBNA-1-Befund vor; aufgrund der anderen Marker muß dann zwischen akuter EBV-Infektion, Anti-EBNA-1-Verlust nach Immunsuppression oder "kein Hinweis auf EBV-Infektion" differenziert werden.

In dem Leuchten aller Zellen innerhalb der Population bei positivem Anti-EBNA-1-Befund liegt jedoch ein Problem, das bisher zuwenig berücksichtigt wurde. So können bis zu 5 % der untersuchten Seren eine anti-nukleäre Reaktivität besitzen, die einen positiven Anti-EBNA-1-Befund vortäuschen kann und damit die Anti-EBNA-1-Bestimmung unsicher werden läßt. Dies ist im Hinblick auf die Bedeutung dieses Markers fatal.

Aufgabe der Erfindung ist es daher, einen für Anti-EBNA-1 spezifischen Immunfluoreszenztest bereitzustellen, mit dem das Vortäuschen eines positiven Anti-EBNA-1-Befunds vermieden wird.

Gelöst wird diese Aufgabe durch ein Verfahren zum spezifischen Nachweis von Antikörpern gegen EBNA-1 im Serum mittels Antikomplement-Immunfluoreszenz, indem zu EBNA-1 exprimierenden Zellen, die eine Deletion des EBNA-2 Gens tragen, ein Überschuß EBV-negativer Humanzellen hinzugefügt und dieses Gemisch mit dem zu prüfenden Serum und Komplement behandelt und anschließend mit Fluorescein-markiertem Antikomplement in Kontakt gebracht wird.

Die Grundtechnik orientiert sich an dem Verfahren nach Reedman und Klein. Die Spezifität für die Bestimmung von Antikörpern gegen EBNA-1 wird dadurch erreicht, daß EBV-positive Burkitt-Lymphomzellen verwendet werden, die eine Deletion des EBNA-2 Gens tragen und somit nur EBNA-1 exprimieren können. Dies gilt beispielsweise für die Zellinie P3HR-1 (Bornkamm et al., J. Virol. 1982; 43:952-968). Aus dieser Linie wurde ein Subklon "3/B-8" isoliert, der sich durch extrem niedrige unspezifische Anfärbbarkeit in der Immunfluoreszenz und eine brilliante Immunfluoreszenz der spezifisch positiven Zellen auszeichnet (Kampmann et al.).

Als erfindungsgemäß einzusetzende EBV-negative Zellinie eignet sich beispielsweise die Lymphomlinie BJAB. Damit wird erreicht, daß bei Vorliegen spezifischer Anti-EBNA-1-Antikörper ein Muster von positiven Zellen auf einem Hintergrund von negativen Zellen in der Immunfluoreszenz erkannt wird. Alle spezifisch positiven Seren müssen exakt das gleiche Muster ergeben. Seren, die im klassischen Anti-EBNA-1-Test ein Leuchten hervorrufen, das vom spezifischen Anti-EBNA-Befund naturgemäß nicht unterschieden werden kann, werden in dem neuen Test sofort erkannt, da sie alle Zellen des Gemischs anfärben.

Ist der Titer der antinukleären nicht EBV-spezifischen Antikörper kleiner als der eventuell vorkommender spezifischer Anti-EBNA-1-Antikörper in dem Serum, wird gemäß einer bevorzugten Ausführungsform der Erfindung nach Titration der unspezifische Hintergrund verschwinden und das EBNA-1 spezifische Muster auftauchen. Der unspezifische Titer ist meist nicht höher als 1:32, EBNA-1 läßt sich auf diesem Wege im Mittel bis zu einem Titer von 1:256 eindeutig nachweisen.

Sind unspezifischer und spezifischer Titer gleich hoch oder ist der unspezifische Titer höher als der spezifische, kann natürlich keine Aussage über den EBNA-1-Status gemacht werden. Doch liegt der diagnostisch immense Vorteil des neuen Verfahrens auch darin, daß das Vorliegen eines Problemserums erkennbar wird und damit kein falscher Ausschluß einer akuten EBV-Infektion durch einen falschpositiven Anti-EBNA-1-Befund erfolgt.

Der neue Anti-EBNA-1-Test bestimmt also spezifisch Antikörper gegen EBNA-1 und stellt im gleichen Untersuchungsgang sicher, daß kein falschpositiver Befund aufgrund einer antinukleären, nicht EBV-spezifischen Reaktion vorliegt. Er kann daher mit hoher Spezifität den für die EBV-Serologie zentralen Marker Anti-EBNA-1 bestimmen.

Der Überschuß der erfindungsgemäß zu verwendenden EBV-negativen Humanzellen kann 55 %, bezogen auf die Zellanzahl, betragen, oder aber auch darüber liegen, beispielweise bei 60, 70, 75 oder 80%.

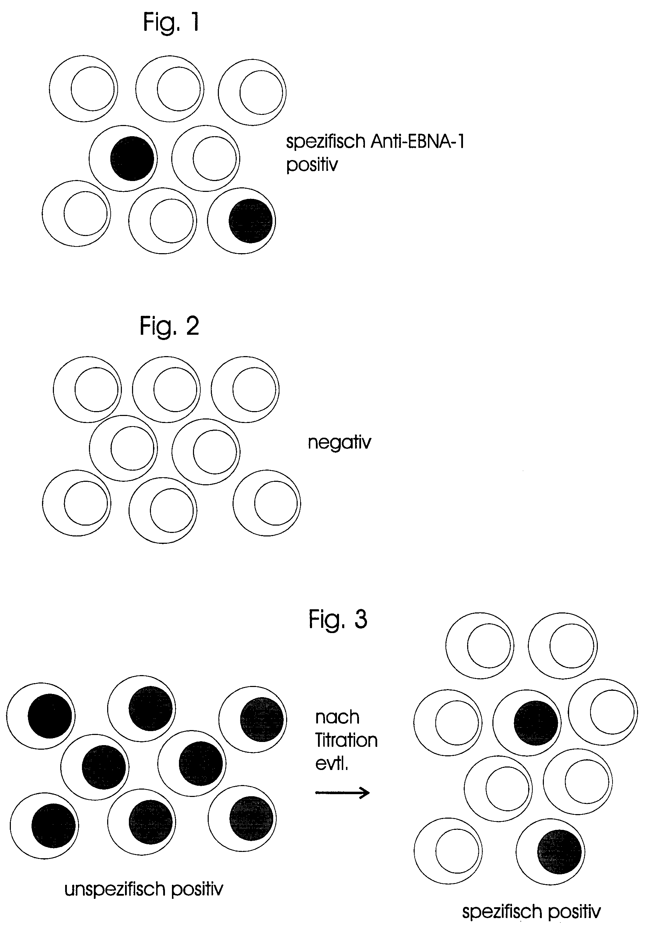

- Fig. 1: zeigt eine zeichnerische Abbildung eines spezifisch anti-EBNA-1 positiven Tests.
- Fig. 2: zeigt die zeichnerische Abbildung eines negativen Tests.
- Fig. 3: zeigt die zeichnerischen Abbildungen eines unspezifischen Tests und diejenige nach Titration, die ein spezifisch positives Ergebnis liefert.

Die zur Durchführung des erfindungsgemäßen Verfahrens erforderlichen Reagenzien lassen sich in einem Testkit zusammenstellen und umfassen insbesondere Zellen eines EBV-positven Klons und einer EBV-negativen Zellinie.

### BEISPIEL

Zur Durchführung des erfindungsgemäßen Verfahrens werden Zellen eines EBV-positiven Klons 3/B-8 und der EBV negativen Zellinie BJAB zentrifugiert und in PBS aufgenommen. Ein Teil 3/B-8 wird mit 4 Teilen BJAB vermischt und auf einen Objektträger in der Weise aufgebracht, daß ein fast zusammenhängender Rasen erhalten wird. Man läß die Suspension antrocknen und fixiert bei -20°C in Methanol 7 Min. lang. Diese Präparationen werden getrocknet und in verschlossenen Aluminiumbeuteln aufbewahrt.

Die gewünschte Serumverdünnung wird hergestellt und auf das fixierte Zellgemisch aufgetropft. Es wird etwa 1 Stunde bei 37°C inkubiert und anschließend in PBS gewaschen. Humankomplement wird zugegeben und ein weiteres Mal 1 Stunde lang bei 37°C inkubiert. Es folgt ein weiteres Waschen und Zugabe von mit Fluorescein markiertem Antikomplement und anschließende einstündige Inkubation. Dann werden die Proben gewaschen und nach bekannten Verfahren eingedeckelt.

Im Fluoreszenzmikroskop mit für Fluorescein geeigneten Filtern und entsprechender Anregung und mindestens 400facher Vergrößerung werden die Proben untersucht und wie folgt bewertet.
1. 25 % der Zellen zeigen spezifische nukleäre Fluoreszenz, d.h. positiver Anti-EBNA-1-Befund und sicherer Ausschluß einer EBV-Infektion.
2. Keine leuchtenden Zellen oder nur vereinzelt leuchtende Zellen, letzteres aufgrund einer VCA-Fluoreszenz, d.h. negativer Anti-EBNA-1-Befund. Weitere Diagnostik aufgrund des VCA-IgG und dessen Avidität in der klassischen indirekten Immunfluoreszenz.
3. Mehr als 25 % der Zellen zeigen Signal, d.h. Hinweis auf das Vorliegen antinukleärer Antikörper. Die weitere Titration wird zeigen, ob der unspezifische Befund von einem spezifischen Anti-EBNA-1-Befund differenziert werden kann, sofern der Titer des letzteren höher ist als der unspezifische. In jedem Fall werden jedoch Problemfälle erkannt und falsche Ausschlüsse akuter EBV-Infektionen vermieden.

## Patentansprüche

1. Verfahren zum spezifischen Nachweis von Antikörpern gegen EBNA-1 im Serum mittels Antikomplement-Immunfluoreszenz, indem EBNA-1 exprimierenden Zellen, die eine Deletion des EBNA-2-Gens tragen, mit dem zu prüfenden Serum und Komplement behandelt und anschließend mit Fluorescein-markiertem Antikomplement in Kontakt gebracht wird, **dadurch gekennzeichnet,** daß man diesem Gemisch einen Überschuß EBV negativer Humanzellen hinzufügt.

2. Verfahren nach Anspruch 1, **dadurch gekennzeichnet**, daß man Verdünnungen des zu prüfenden Serums anfertigt und diese in der angegebenen Weise behandelt und auswertet.
